# EUROPEAN PATENT SPECIFICATION

(11) **EP 2 793 769 B1**
(45) Date of publication and mention of the grant of the patent: **18.04.2018**
(21) Application number: 12859389.4
(22) Date of filing: 18.12.2012
(51) Int. Cl.: A61F 6/04, A61F 6/02

(54) **CONDOM HAVING A RAISED CONTOURED SURFACE AND METHOD AND APPARATUS FOR MANUFACTURING**
KONDOM MIT HOHER KONTURIERTER OBERFLÄCHE SOWIE VERFAHREN UND VORRICHTUNG ZUR HERSTELLUNG
PRÉSERVATIF PRÉSENTANT UNE SURFACE SURÉLEVÉE EN DÉNIVELÉ ET PROCÉDÉ ET APPAREIL UTILISABLES EN VUE DE SA FABRICATION

(30) Priority: 19.12.2011 US 201161577509 P
(43) Date of publication of application: 29.10.2014
(73) Proprietor: SxWell Australia Pty. Ltd., Richmond, VIC 3121 (AU)
(72) Inventor: NGUYEN, KC, Dothan, Alabama 36303 (US); CHUAH, Beng Sim, Selangor, 47301 (MY); KUNG, Adeline Ai Lin, Selangor, 47800 (MY); GROSKORTH, Matthew Steven, Prahan, Vic., Australian Capital Territory 3181 (AU); PONGTHANOMSAK, Chayaporn, Surat Thani, 84130 (TH)
(74) Representative: Zimmermann & Partner Patentanwälte mbB
(86) International application number: PCT/US2012/070316
(87) International publication number: WO 2013/096293

(56) References cited:
- GB-A- 2 100 988
- GB-A- 2 220 358
- US-A- 4 852 586
- US-A1- 2004 000 315
- US-A1- 2004 099 274
- US-A1- 2004 099 274
- US-A1- 2006 048 784
- US-A1- 2011 073 117
- US-B1- 6 182 661

## Description

### BACKGROUND

### Field of the Invention

Embodiments of the present invention generally relate to condoms and, more particularly, to a condom having raised features and surface textures in various designs and patterns, and condom formers, and methods and apparatus for making such condoms.

### Description of the Related Art

Condoms and similar prophylactic/protective devices provide physical barriers against the transmission of bodily and other fluids. Condoms are typically made of an elastomeric layer. Condom designs include a shaft portion having an open end and a closed end, which terminates in a tip.

Designers have attempted to create condoms having textures and patterns to provide stimulation and enhanced aesthetics. For example, previous condoms have studs or ribs. Some condoms have been textured by heat- and pressure-embossing various features onto films, and subsequently forming the condom. However, a condom of this structure requires several additional manufacturing steps. Additionally, past condoms are limited in that their structures have lesser raised surface areas compared with the total surface area of the condom.

Consequently, a condom providing protection against the transmission of bodily fluids and other fluids, which includes a plurality of raised features having a total surface area larger the total surface area of the base surface, and improved methods and apparatus for manufacturing such condoms, wherein the raised features are integrally formed therewith from a single operation, as well as improved aesthetics, would present a significant advance in the art. GB 2 100 988 A relates to a contraceptive device formed of rubber or the like having an outer semen pouch with two medially located leakage preventing portions of a lesser diameter than that of the body of the device and spaced surface areas covered with a large number of small protrusions each having an indentation in its end. GB 2 220 358 A relates to a contraception and flavor delivery system including a prophylactic element for at least partial insertion into a bodily orifice defining an extended tubular member. US 2011/073117 A1 relates to a condom, including a non-porous sheath member, having an open end and a rounded end, and shaped to cover the glans and body portion of a penis, and at least one rib having at least one outer portion and at least one inner portion, wherein the at least one outer portion is attachedly fixed to said non-porous sheath member. US 2004/099274 A1 relates to a condom including a body that has a wall, a closed end and an open end. The wall of the condom has an interior surface and an exterior surface. A textured portion on the condom functions to increase the sensation transmitted by the condom during use. The textured portion may be positioned in a curved region of the condom. When used as a female condom, the textured portion inhibits the possibility of the condom be displaced.

### SUMMARY

Condoms, condom formers, and methods for forming condoms in accordance with the present invention, substantially as shown in and/or described in connection with at least one of the figures, as set forth more completely in the claims, are disclosed. Various advantages, aspects, and novel features of the present disclosure, as well as details of exemplary embodiments thereof, will be more fully understood from the following description and drawings.

### BRIEF DESCRIPTION OF THE DRAWINGS

So that the manner in which the above recited features of the present invention can be understood in detail, a more particular description of the invention, briefly summarized above, may be had by reference to embodiments, some of which are illustrated in the appended drawings. It is to be noted, however, that the appended drawings illustrate only illustrative embodiments of this invention and are therefore not to be considered limiting of its scope, for the invention may admit to other equally effective embodiments.
FIG. 1 depicts a condom having a pattern of raised features, according to embodiments of the invention;
FIG. 2 depicts a portion of a second pattern of raised features for a condom, according to embodiments of the invention;
FIG. 3 depicts a portion of a third pattern of raised features for a condom, according to embodiments of the invention;
FIG. 4 depicts a fourth pattern of raised features for a condom, according to embodiments of the invention;
FIG. 5 depicts an example of a pattern of diamond-shaped raised features for a condom, according to embodiments of the invention;
FIG. 6 depicts a cross sectional view of FIG. 4 or FIG. 5 taken along line 6-6 of FIG. 4 or 5, according to embodiments of the invention;
FIG. 7 depicts a condom having a pattern of hexagonally-shaped raised features on a condom, according to embodiments of the invention;
FIG. 8 depicts a former having a pattern of raised features on a condom, according to embodiments of the invention; and
FIG. 9 is a flow diagram of a typical method of the condom forming process according to some embodiments of the inventions.

To facilitate understanding, identical reference numerals have been used, where possible, to designate comparable elements that are common to the figures. The figures are not drawn to scale and may be simplified for clarity. It is contemplated that elements and features of one embodiment may be beneficially incorporated in other embodiments without further recitation.

### DETAILED DESCRIPTION

Embodiments of the present invention pertain to a condom having a tubular sheath and a plurality of raised features disposed thereon in which the total top surface area of the raised features is greater than the total surface area of the non-raised surface area of the condom. In some embodiments, the plurality of raised features may form patterns on the tubular sheath, which may be in a direction parallel or perpendicular to at least a portion of the tubular sheath. The plurality of raised features disposed on the tubular sheath may comprise a structure adapted to stimulate the areas of heightened nerve stimulation found on both the male and female body, for example, the frenulum, the vagina-labium minor interface, and the clitoris, for example. In some embodiments, condom patterns may comprise raised features, which when viewed from a top plan perspective may be in the shape of circles, ovals, irregularly-shaped polygons or regularly-shaped square or rectangular diamonds. Some patterns, when viewed from a top plan perspective may appear as a snakeskin pattern.

Embodiments of the invention may further comprise walls and other protrusions to provide additional features within the plurality of raised features disposed on a condom. Furthermore, the raised features impart additional contrast to the appearance of the condom. The walls and protrusions may be incorporated around the edges of individual raised features and are integrally formed thereon. The structure and disposition of the plurality of raised features, walls, or protrusions is unexpected in such condoms because they are integrally formed on condoms using dipping processes, wherein a former comprising the desired texture is dipped into a bath of a latex composition.

Condoms formed by dipping a mold or former into an elastomeric emulsion or latex, dried, cured, and stripped from the mold as is known in the art are contemplated herein. As defined herein, "integrally formed" means a structure formed in a single operation and which cannot be dissembled. For example, raised features integrally formed within a condom indicates that the raised features are formed during the same single operation, here, the dipping of a former into a tank of latex emulsion, as all other parts of the condom

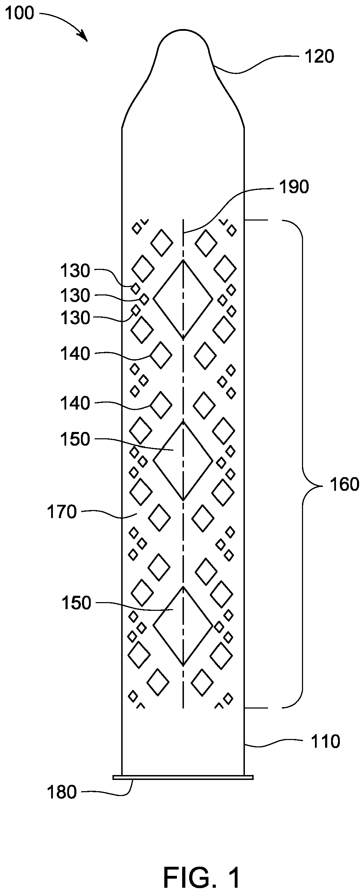

FIG. 1 depicts a condom having a pattern of raised features, according to embodiments of the invention. Condoms include an open end 110, a closed teat end 120, tubular sheath 160 extending therebetween, raised features 130, 140, 150, and a surface defining a base area 170. All condoms in accordance with all embodiments of the present invention disclosed herein optionally comprise roll ring 180, as shown on condom 100. In the pattern of this embodiment, a longitudinal axis 190 extends through raised features 150, thereby illustrating a longitudinal pattern of the raised features. Condom 100 also includes an axial length and a surface defining a circumference, the circumference being approximately 110 mm in some embodiments. In one or more embodiments, tubular sheath 130 has a thickness in the range from about 0.030 mm to about 0.200 mm, including the height of the plurality of raised features 130, 140, 150 disposed thereon. In one or more embodiments, tubular sheath 160 has a thickness in the range from about 0.040 mm to about 0.120 mm, including the height of raised features 130, 140, 150 disposed thereon. In one or more embodiments, the thickness of the tubular sheath increases from open end 110 to closed end 120.

Raised features 130, 140, 150 may be in any of several geometric shapes, such as square or rectangular diamonds or, as discussed below, circular or oval. Raised features 130, 140, 150 may comprise different sizes within the same condom 100. For example, in some embodiments, as shown in FIG. 1, the raised features are diamond shaped, where raised features 130 range in size from approximately 1.0-3.5 x 1.0-3.5 x 1.0-3.5 x 1.0-3.5 x 1.0-2.0 mm, raised features 140 range in size from approximately 4.0-10.5 x 4.0-10.5 x 4.0-10.5 x 4.0-10.5 mm, and raised features 150 range in size from approximately 11.0-14.0 x 11.0-14.0 x 11.0-14.0 x 11.0-14.0 mm.

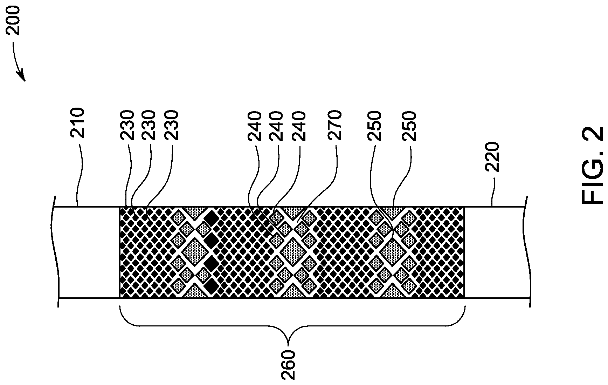

FIG. 2 depicts a portion of a second pattern of raised features for a condom, according to embodiments of the invention. FIG. 2 shows condom pattern 200, which may be imparted to a condom, between an open end 210 and a teat end 220 (condom not fully illustrated for ease of presentation). Disposed therebetween is tubular sheath 260, which comprises a base surface 270 and raised features 230, 240, 250. In embodiments in accordance with the pattern, raised features 230, 240, 250, which may be similar in size and shape as raised features 130, 140, 150, traverse the condom laterally. In other words, patterns in this embodiment have raised features disposed in ring-like structures around the circumference of the condom, providing a certain appearance.

In FIG. 2, raised features 250 show a darker shade than raised features 240, and raised features 240 are darker than raised features 230. The darker colors indicate the height of the raised features on the surface of the condom with respect to each other. In other words, the surface of raised features 250 are further from base surface 270 of the condom where no raised features are disposed, such as at open end 210 or teat end 220. Also, as will be discussed below, raised features 250 may have a different height and/or surface texture than raised features 230, 240. Similarly, raised features 240 are higher from the base surface 270 of the condom, though not as high as raised features 250. The lateral distance between each of the raised features ranges from 0.05-4.0 mm. In some embodiments, the distance between the raised features may be 1-2 mm. Varying the size, shape, and texture of the raised features disposed on condoms formed in accordance with embodiments of the invention can impart different patterns presenting different appearances when viewed from a plan perspective.

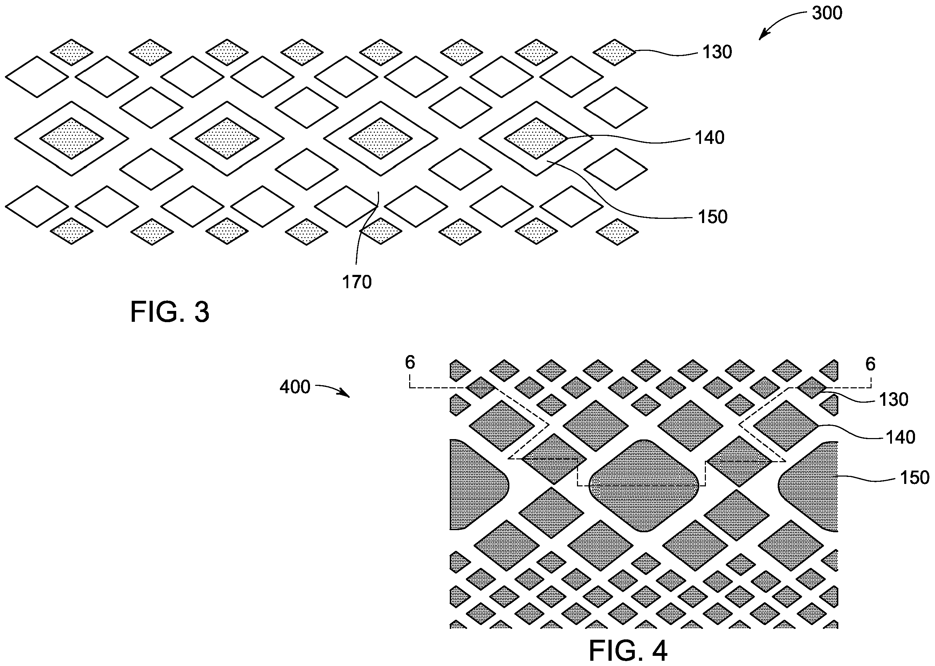

FIG. 3 depicts a portion of a third pattern of raised features for a condom, according to embodiments of the invention. Pattern 300 has diamond-shaped raised features 130, 140, 150 and base surface 170 disposed therebetween. The area of diamond-shaped member 150 may optionally comprise an area substantially equal to the area of diamond-shaped member 140. As can be seen, raised feature 140 is disposed on top of raised feature 150. Raised features 130 and 140 may have a surface texture different than raised feature 150. This difference in surface texture yields additional contrast to the pattern. In some embodiments, different patterns of surface textures can be incorporated around the edges of each raised feature to accentuate the texture contrast.

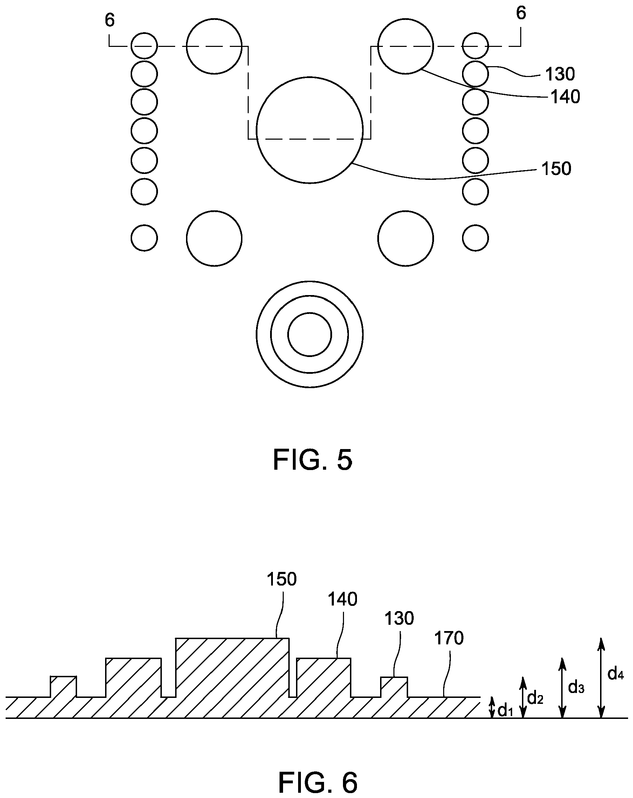

FIG. 4 depicts a fourth pattern of raised features for a condom, according to embodiments of the invention. Pattern 400 has diamond-shaped raised features 130, 140, 150 and base surface 170 disposed therebetween. In some embodiments of the invention, the raised features comprise any geometrical shape. FIG. 5 depicts a pattern of circular-shaped raised features for a condom, according to embodiments of the invention. This pattern shows fewer raised features 130, 140, 150 for purposes of clarity. Both FIGS. 4 and 5 have cross section 6-6 drawn through it. As shown in FIG. 6, base surface 170 may be at height d1, raised features 130 may be at height d2, raised features 140 may be at height d3, and raised features 150 may be at height d4. Nonetheless, it can be seen that cross-section 6-6 of the raised features are substantially similar in the embodiments of the invention for FIGS. 4 and 5. Furthermore, the different heights of raised features 130, 140, 150 may impart a certain contrast to the appearance of the condoms from a top plan perspective, as well as a tactile difference during use. There may be a difference in height of approximately 0.05 mm between each of d1, d2, d3, and d4, though the height difference may range between approximately 0.1 and 0.7 mm. In alternative embodiments, the heights d2, d3, and d4 of raised features 130, 140, 150 may be the same. In other embodiments, the height d2 > d3 > d4 of raised features 130, 140, 150. Other embodiments may comprise where d2 > d3 = d4, d2 = d3 > d4 and d2< d3 = d4.

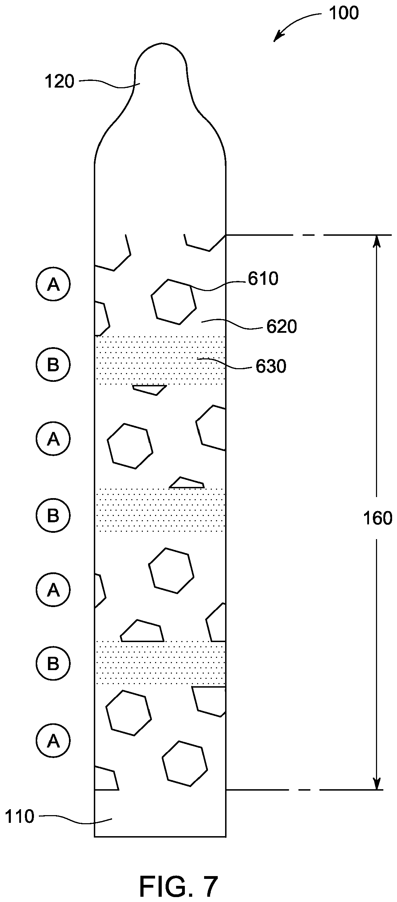

FIG. 7 depicts a condom having a pattern of hexagonally-shaped raised features disposed thereon, according to embodiments of the invention. In some embodiments, raised features may be disposed on substantially all of a condom, while in other embodiments, open end 110 and teat end 120 of condom 100 are free from raised features, i.e., condom 100 may have raised features disposed only on tubular sheath 160. For ease of presentation, only a few raised features 610 are shown. However, it is to be understood that other raised features of differing lengths, widths, and thickness could be disposed thereon. In some embodiments, condom 100 is separated into six sections of areas having alternating portions A and B. The A sections comprise sections having raised features 610 disposed thereon while the B sections do not have raised features. Condom 100 can further comprise where, as discussed below, the A sections have surface textures consistent with that of a former that is acid-etched and the B sections have surface textures 630 consistent with that where the former is sandblasted. In these alternating sections, tubular sheath 160 comprises A sections having raised features for about 20-60 mm in length and B sections about 20-60 mm in length, while in other embodiments, this distance may be up to 30 mm in length or greater.

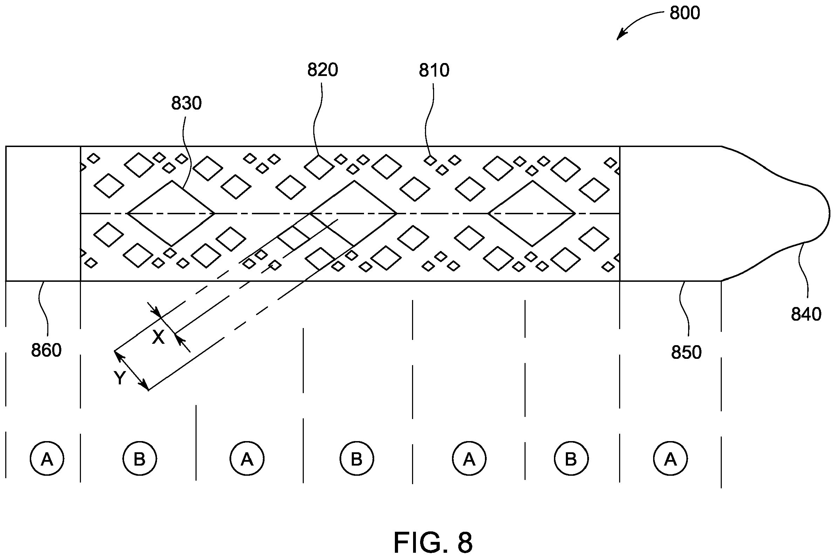

FIG. 8 depicts a condom former It is to be understood that condoms formed with a former become the negative of the former and therefore, the features of the former are imparted to a condom made therewith. For example, former 800 may comprise diamond-shaped members of three different sizes or more. Former 800 has small diamond-shaped members 810, medium diamond-shaped members 820, and large diamond-shaped members 830. Former 800 will be 300 mm long, in other embodiments, the former will be 330 mm long, and it other embodiments, the former may be 360 mm long or longer. Former 800 will be approximately 30 mm in diameter, and in some embodiments, the former may be about 35 mm in diameter or wider. Former 800 further comprises teat end 840, middle portion 850, and end 860.

The materials former 800 comprise glass, borosilicates, or other ceramics. The depressions may on the surface of the former be imparted by sandblasting, such as via a Grade 120 sand or other appropriate grades, such as 30, 60, 80, 150, 180. Each grit size can yield a different depth on the glass surface. Acid-etching, diamond cutting, or a combination of any or all with sandblasting can yield a plurality of uneven surfaces and textures on the mold. These surface designs and textures provide different patterns to be disposed thereon.

The depressions on former 800 may comprise several different patterns, and be in different sizes, shapes, and depths. As shown in FIG. 7, the former has diamond-shaped members that may have dimensions ranging in size from, for example and as discussed above with respect to the condoms, 1.0 mm per side to 12.0 mm per side. Sides of small diamond-shaped members 810 may range in size from approximately 1.0-3.5 x 1.0-3.5 mm. Sides of medium diamond-shaped members 820 may range in size, as shown by dimension X, from 4.0-10.5 x 4.0-10.5 mm. Sides of large diamond-shaped members may range in size, as shown by dimension Y, from 11.0-14.0 x 11.0-14.0 mm. The depth of the diamond-shaped members 810, 820, 830 ranges from approximately 0.05 to 1.5 mm.

Former 800 may comprise where all surfaces are acid-etched, all surfaces are sandblasted, and where large-diamond shaped members 830 are sandblasted and other surfaces are acid-etched, providing different textured surfaces, thereby imparting additional contrast on the formers and the condoms formed therefrom. Acid-etching the depressions within the former, irrespective of shape, increases the depth of the raised feature at the edge. When a former is dipped to make a condom, a raised wall is disposed around the periphery of the raised feature. Furthermore, former 800 may comprise alternating sections having a pattern while leaving other sections free from a pattern as discussed above. For example, former 800 is separated into six or more sections having alternating portions A and B.

The edges of the large and/or medium diamond-shaped members on the former may range from approximately 0.70 to 0.90 mm in width. These grooves, which allow the structure of peripheral walls around the raised features on condoms formed therewith, impart additional contrast to the former and the condoms. Furthermore, protrusions and other raised features may be disposed on condoms of the present invention in accordance with commonly-assigned US Provisional application Serial No. 61/385,694, filed September 23, 2010; and co-pending, commonly-assigned US Patent Application Serial No. 13/243,038, filed September 23, 2011.

Condoms in accordance with the invention may be formed from an elastomeric material that may include an elastomeric rubber. The elastomeric rubber may be a synthetic rubber, natural rubber latex, and combinations, mixtures, or blends thereof. Other examples of suitable elastomeric materials include synthetic polyisoprene, guayule, aqueous and non-aqueous polyurethanes, vinyls, polyethylenes, copolymers, block copolymers, butadienes, and blends, mixtures, or combinations thereof. The term "natural rubber latex" as used in this disclosure encompasses cured elastomeric material sourced from Hevea brasiliensis (the traditional rubber tree), Parthenium argentatum (guayule), sunflower, goldenrod, and the like, as well as genetically modified variations of these or other biological sources. In some embodiments of the invention, the elastomeric material is a synthetic polyisoprene as disclosed in commonly-assigned US Patent No. 8,087,412. Any elastomeric, polymeric, or latex material for forming condoms may comprise additional additives, such as surfactants, curing agents, cross-linkers, and the like to control or modify the physical properties, including thixotropic properties, of the elastomeric, polymeric, or latex emulsion formed therewith. Other examples of additives that may be used in embodiments of the inventions are disclosed in commonly-assigned Patent No. 5,977,223.

Coagulants that may optionally be disposed on the former before dipping into the elastomeric, polymeric, or latex emulsion include ionic coagulants (e.g., mono-, di- and tri-valent cations). Examples of monovalent cationic coagulants include quaternary ammonium salts such as cyclohexylamine salts (e.g., cyclohexylamine acetate). Divalent cationic coagulants, such as alkaline earth metal salts (e.g., calcium salts, calcium chloride) or transition metal salts (e.g., zinc salts), may be used. Also, the nitrates of these salts, such as calcium nitrate, may be used. In addition to conventional coagulants, heat sensitizing agents may also be used. Thus, heat sensitizing agents such as polyether siloxanes may be mixed with the polyurethane dispersion. The amount of coagulant present in the coagulant solution may range from 5 to 60% w/w solution, from 5 to 40% w/w or from 10 to 35% w/w.

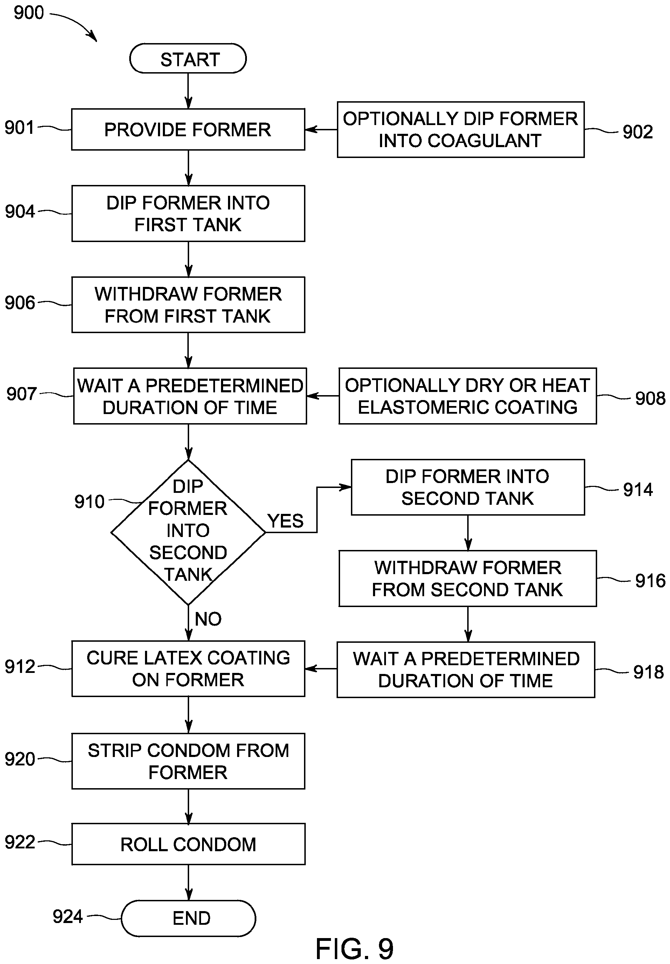

Figure 9 is a flow diagram depicting a typical example of process 900 for forming a condom according to some embodiments of the inventions. The process begins at process block 901 wherein a former is provided, which may be optionally dipped into a coagulant bath at process block 902, and proceeds to process block 904, wherein the former is dipped into a first tank, the first tank containing a latex emulsion. The latex emulsion may be an aqueous mixture of one or any of the natural or synthetic latexes discussed above. At process block 904, a latex coating forms on the former. At process block 906, the former is withdrawn from the first tank. At process block 907, the process waits a predetermined amount of time. At this point, the elastomeric coating disposed on the former may be allowed to dry in ambient air or may be heated at process block 908. Process 900 then proceed to step 912 for curing of the latex condom. The condom is then stripped from the former at process block 920, rolled and/or packaged at process block 922, and the process subsequently terminates at step 924. Alternately, the former may be dipped into the first tank one or more times to build more layers into the condom. In another embodiment, following the step of waiting for a predetermined amount of time at step block 907, the former having a latex coating disposed thereon may be dipped into a second tank containing a similar or different emulsion at step block 914. The former is then withdrawn at step block 916. Process 900 then proceeds to wait a predetermined duration of time at step 918 and to step 912 wherein the coatings of latex is cured. Then condom may then be stripped, rolled, and/or packaged as before.

In some embodiments, the first dip tank may contain a latex emulsion having a relatively light color for example, white, off-white, pearl, and the like, while the second dip tank contains a latex emulsion having a color relatively darker than the emulsion in the first dip tank, for example black, brown, green, and the like. In another embodiment, the color of the first dip tank is darker and the second dip tank is lighter. The incorporation of latexes having different colors imparts additional contrast to the pattern of raised features on the condom. embodiments of the present invention include a packaging containing the condom, as otherwise described herein.
Further embodiments of the present invention include a packaging containing the condom. Examples of packaging include, without limitation, wrappers, boxes, tubs having a sealed enclosure, and combinations thereof. In many embodiments, these packages may be formed from materials substantially resistant to the passage of air and/or moisture, as well as ingredients that may be present on the condom, so that ingredients present on the condom are retained, substantially unchanged, during storage prior to use.

Although some embodiments have been discussed above, other implementations and applications are also within the scope of the following claims. Although the invention herein has been described with reference to particular embodiments, it is to be understood that these embodiments are merely illustrative of the principles and applications of the present invention.

All numerical values recited herein are exemplary, are not to be considered limiting, and include ranges therebetween, and can be inclusive or exclusive of the endpoints. Optional included ranges can be from integer values therebetween, at the order of magnitude recited or the next smaller order of magnitude. For example, if the lower range value is 0.1, optional included endpoints can be 0.2, 0.3, 0.4 ... 1.1, 1.2, and the like, as well as 1, 2, 3 and the like; if the higher range is 10, optional included endpoints can be 7, 6, and the like, as well as 7.9, 7.8, and the like.

## Claims

1. A condom (100), comprising:
an open end (110);
a closed end (120);
a tubular sheath (160) extending from the open end (110) to the closed end (120), comprising:
a tip portion, adapted to cover a glans of a penis, disposed adjacent to the closed end (120);
a plurality of raised features (130, 140, 150) disposed on the tubular sheath (160), having a top surface area and a base surface area,
**characterised in that**
the total raised top surface area is greater than the total base surface area on the condom (100).

2. The condom (100) of claim 1, wherein the plurality of raised features (130, 140, 150) are of different sizes.

3. The condom (100) of claim 2, wherein the plurality of raised features (130, 140, 150) are of different shapes.

4. The condom (100) of claim 3, wherein the raised features (130, 140, 150) are square-, rectangular-, oval-, circular-shaped, or diamond-shaped.

5. The condom (100) of claim 4, wherein the sides or axes of the raised features (130, 140, 150) range in size from 1.0 to 14.0 mm and from a height between 0.05 to 1.5 mm.

6. The condom (100) of claim 1, wherein a plurality of shapes form a pattern.

7. The condom (100) of claim 6, wherein the pattern is parallel or perpendicular to the longitudinal axis (190) of the condom 100.

8. The condom (100) of claim 1, wherein at least some of the plurality of raised features (130, 140, 150) are diamond-shaped.

9. The condom (100) of claim 8, wherein at least some of the plurality of diamond-shaped raised features (130, 140, 150) are of different height.

10. The condom (100) of claim 9, wherein a first plurality of diamond-shaped raised features has a height which is greater than a second plurality of diamond-shaped raised features, and wherein the first plurality of diamond-shaped raised features have a colour which is darker than the colour of the second plurality of diamond-shaped raised features.

11. The condom (100) of claim 10, wherein the color is black, brown or green.

12. The condom (100) of claim 8, wherein at least some of the plurality of diamond-shaped raised features (130, 140, 150) are of at least one of a different height, a different size, a different shape or a different texture.

## Patentansprüche

1. Kondom (100), umfassend:
ein offenes Ende (110);
ein geschlossenes Ende (120);
eine rohrförmige Hülle (160), die sich vom offenen Ende (110) zum geschlossenen Ende (120) erstreckt, umfassend:
einen Spitzenabschnitt, der dazu angepasst ist, eine Glans eines Penis zu bedecken und angrenzend an das geschlossene Ende (120) angeordnet ist;
mehrere an der rohrförmigen Hülle (160) angeordnete erhöhte Ausstattungen (130, 140, 150) mit einem Oberflächenbereich und einem Grundflächenbereich,
**dadurch gekennzeichnet, dass**
der gesamte erhöhte Oberflächenbereich am Kondom (100) größer ist als der gesamte Grundflächenbereich.

2. Kondom (100) nach Anspruch 1, wobei die mehreren erhöhten Ausstattungen (130, 140, 150) von verschiedenen Größen sind.

3. Kondom (100) nach Anspruch 2, wobei die mehreren erhöhten Ausstattungen (130, 140, 150) von verschiedenen Formen sind.

4. Kondom (100) nach Anspruch 3, wobei die erhöhten Ausstattungen (130, 140, 150) quadratisch, rechteckig, oval, kreisförmig oder rautenförmig sind.

5. Kondom (100) nach Anspruch 4, wobei die Seiten oder Achsen der erhöhten Ausstattungen (130, 140, 150) in der Größe von 1,0 bis 14,0 mm und von einer Höhe zwischen 0,05 bis 1,5 mm reichen.

6. Kondom (100) nach Anspruch 1, wobei mehrere Formen ein Muster bilden.

7. Kondom (100) nach Anspruch 6, wobei das Muster parallel oder senkrecht zur Längsachse (190) des Kondoms (100) ist.

8. Kondom (100) nach Anspruch 1, wobei zumindest einige der mehreren erhöhten Ausstattungen (130, 140, 150) rautenförmig sind.

9. Kondom (100) nach Anspruch 8, wobei zumindest einige der mehreren rautenförmigen erhöhten Ausstattungen (130, 140, 150) von verschiedener Höhe sind.

10. Kondom (100) nach Anspruch 9, wobei eine erste Mehrzahl rautenförmiger erhöhter Ausstattungen eine Höhe hat, die größer ist als eine zweite Mehrzahl rautenförmiger erhöhter Ausstattungen, und wobei die erste Mehrzahl rautenförmiger erhöhter Ausstattungen eine Farbe hat, die dunkler ist als die Farbe der zweiten Mehrzahl rautenförmiger erhöhter Ausstattungen.

11. Kondom (100) nach Anspruch 10, wobei die Farbe schwarz, braun oder grün ist.

12. Kondom (100) nach Anspruch 8, wobei zumindest einige der mehreren rautenförmigen erhöhten Ausstattungen (130, 140, 150) zumindest von einer unterschiedlichen Höhe, einer unterschiedlichen Größe, einer unterschiedlichen Form oder einer unterschiedlichen Textur sind.

## Revendications

1. Préservatif (100), comprenant :
une extrémité ouverte (110) ;
une extrémité fermée (120) ;
une gaine tubulaire (160) s'étendant de l'extrémité ouverte (110) à l'extrémité fermée (120), comprenant :
une partie de bout, adaptée pour recouvrir un gland d'un pénis, disposée au voisinage de l'extrémité fermée (120) ;
une pluralité de formes en relief (130, 140, 150) disposées sur la gaine tubulaire (160), ayant une aire de surface supérieure et une aire de surface de base,
**caractérisé en ce que**
l'aire totale de surface supérieure en relief est supérieure à l'aire totale de surface de base sur le préservatif (100).

2. Préservatif (100) selon la revendication 1, dans lequel les formes de la pluralité de formes en relief (130, 140, 150) sont de différentes tailles.

3. Préservatif (100) selon la revendication 2, dans lequel les formes de la pluralité de formes en relief (130, 140, 150) sont de différentes formes.

4. Préservatif (100) selon la revendication 3, dans lequel les formes en relief (130, 140, 150) sont de forme carrée, rectangulaire, ovale, circulaire ou en losange.

5. Préservatif (100) selon la revendication 4, dans lequel les côtés ou axes des formes en relief (130, 140, 150) varient en taille de 1,0 à 14,0 mm et à partir d'une hauteur entre 0,05 et 1,5 mm.

6. Préservatif (100) selon la revendication 1, dans lequel une pluralité de formes forme un motif.

7. Préservatif (100) selon la revendication 6, dans lequel le motif est parallèle ou perpendiculaire à l'axe longitudinal (190) du préservatif (100).

8. Préservatif (100) selon la revendication 1, dans lequel au moins certaines de la pluralité de formes en relief (130, 140, 150) sont en forme de losange.

9. Préservatif (100) selon la revendication 8, dans lequel au moins certaines de la pluralité de formes en relief en forme de losange (130, 140, 150) sont de différentes hauteurs.

10. Préservatif (100) selon la revendication 9, dans lequel une première pluralité de formes en relief en forme de losange a une hauteur qui est plus grande qu'une seconde pluralité de formes en relief en forme de losange, et dans lequel la première pluralité de formes en relief en forme de losange a une couleur qui est plus sombre que la couleur de la seconde pluralité de formes en relief en forme de losange.

11. Préservatif (100) selon la revendication 10, dans lequel la couleur est noire, marron ou verte.

12. Préservatif (100) selon la revendication 8, dans lequel au moins certaines de la pluralité de formes en relief en forme de losange (130, 140, 150) ont au moins une parmi une hauteur différente, une taille différente, une forme différente ou une texture différente.
